# EUROPEAN PATENT SPECIFICATION

(11) **EP 3 749 775 B1**
(45) Date of publication and mention of the grant of the patent: **07.06.2023**
(21) Application number: 19702466.4
(22) Date of filing: 07.02.2019
(51) Int. Cl.: C12Q 1/04, G01N 33/569

(54) **IMPROVED METHOD FOR MEASUREMENT OF MICROBIAL BIOMASS**
VERBESSERTES VERFAHREN ZUR MESSUNG VON MIKROBIELLER BIOMASSE
PROCÉDÉ AMÉLIORÉ DE MESURE DE BIOMASSE MICROBIENNE

(30) Priority: 07.02.2018 EP 18155604; 10.04.2018 EP 18166640
(43) Date of publication of application: 16.12.2020
(73) Proprietor: Mycometer A/S, 2970 Hørsholm (DK)
(72) Inventor: REESLEV, Morten, 2970 Hørsholm (DK)
(74) Representative: Inspicos P/S
(86) International application number: PCT/EP2019/052979
(87) International publication number: WO 2019/154898

(56) References cited:
- EP-A1- 2 407 549
- WO-A1-2005/083109
- WO-A1-2010/119270
- WO-A1-2011/103144
- WO-A1-2017/053931
- WO-A2-2009/007719
- US-B1- 6 921 635
- TARENTINO A L ET AL: "Multiple forms of a highly purified @b-N-acetylhexosaminidase from hen oviduct", ARCHIVES OF BIOCHEMISTRY AND BIOPHYSICS, ACADEMIC PRESS, US, vol. 147, no. 2, 1 December 1971 (1971-12-01), pages 446-456, XP024808146, ISSN: 0003-9861, DOI: 10.1016/0003-9861(71)90400-0 [retrieved on 1971-12-01]
- RENAUD LE ONARD ET AL: "The Drosophila fused lobes Gene Encodes anN-Acetylglucosaminidase Involved in N-Glycan Processing", THE JOURNAL OF BIOLOGICAL CHEMISTRY,, vol. 281, no. 8, 24 February 2006 (2006-02-24), pages 4867-4875, XP002798165,
- OIKAWA A ET AL: "Purification and characterization of @b-N-acetylhexosaminidase from maize seedlings", JOURNAL OF PLANT PHYSIOLOGY, ELSEVIER, AMSTERDAM, NL, vol. 160, no. 9, 1 January 2003 (2003-01-01), pages 991-999, XP004955534, ISSN: 0176-1617, DOI: 10.1078/0176-1617-01089

## Description

### FIELD OF THE INVENTION

The present invention relates to detection of fungi such as moulds. More specifically, the present invention relates to improved methods of detection of fungi, where the signal to noise ratio in the detected signal is considerably improved.

### BACKGROUND OF THE INVENTION

International patent publication WO 98/033934 describes a method for quantifying fungal biomass by measuring the level of activity of β-N-acetylhexosaminidase 3.2.1.52 (in the following termed "NAHA") in a sample. NAHA was found to be present in both hyphae and spores and with measurable activities. Further, NAHA activity was shown to correlate well with fungal biomass in different types of environmental samples.

Measurement of NAHA activity as a marker of fungal biomass in environmental samples can be rendered rapid, providing results over the course of minutes rather than days, which is otherwise typical when traditional cultivation methods are used.

When measuring fungi as colony forming units (CFU) in culture media based methods, one spore will in principle give rise to one single colony whereas a mycelium sample, which by nature contains multiple cells, will also produce one single colony and therefore also count as one spore. This inherent underrepresentation of the hyphal growth form compared to the spores is serious, as growth of filamentous fungi consists of approximately 95-100% hyphae. NAHA activity is however present in both hyphae and spores and in approximately the same level per biomass unit. This means that quantification of NAHA activity provides for a much more accurate representative measure of the total fungal biomass (*i*.*e*. the number or concentration of single cells); in other words, the correlation between NAHA activity and biomass of fungi is better than the correlation between CFU and biomass of fungi.

NAHA activity is present in high amounts in all filamentous fungi, but as with almost any other enzymatic activity it is not unique to fungi. Hence, using NAHA activity as a means for quantification of fungi can under certain circumstances lead to pronounced overestimation of fungal biomass due to measured non-fungal NAHA activity.

This is e.g. the case if samples include house dust, which apart from fungal spores may contain pollen, pet dander, human skin cells and house dust mites, all of which exhibit some degree of NAHA activity.

Likewise, sampling from surfaces of plants or other surfaces where non-fungal NAHA activity may be present presents the same risks of overestimation of fungal NAHA activity.

Detection of fungi in blood samples is another example. Human blood contains NAHA activity and being able to selectively inhibit Human NAHA activity could allow detection of the presence of fungal NAHA activity and therefore enable a diagnostic screening tool for mycoses.

Similar problems are likely to arise when determining other specific groups or species of microorganisms based on enzymatic activity. The moment it is not merely of interest to determine the total amount of microbial biomass in a sample, measured activity from similar, homologous or identical enzyme(s) to the one selected as surrogate for the biomass subset of interest will constitute "noise" in the measured signal and can give rise to substantial amounts of false positive results.

There is hence a need to be able to reduce competing signals from "irrelevant" microorganisms when performing measurements in environmental samples corresponding to the measurements disclosed in WO 98/033934 and also the measurements disclosed in WO 2005/083109.

US 6 921 635 B1 discloses methods for identifying yeast using a NAHA reaction in the presence of an inhibitor.

### OBJECT OF THE INVENTION

It is an object of embodiments of the invention to provide improvements in determination of fungal cells (in particular in the environment) based on enzymatic activity of the fungal cells present in a sample

### SUMMARY OF THE INVENTION

The current invention is based on findings made in investigations focused on identifying enzyme inhibitors that could selectively inhibit NAHA from non-fungal sources in systems where fungi (typically moulds) are to be determined.

The activity of enzymes can be inhibited competitively or non-competitively by a large number of inhibitors. Working with different types of inhibitors, the inventors identified different types of enzyme inhibitors that in the experimental setup exhibited very little inhibition of NAHA activity from fungi, but a surprisingly strong inhibition of NAHA activity from a number of non-fungal sources.

It was surprising to find that both specific inhibitors of NAHA activity as well as general inhibitors of enzyme activity exerted the same selective effect, i.e. the ability to preferentially inhibit the NAHA activity in cells/organisms that would produce false positive results in a determination of fungal cells based on NAHA measurements.

Metal ions like Ag⁺, Hg⁺ and Cu²⁺, as well as a more specific NAHA inhibitor, the azasugar 2-Acetamido-1,2-dideoxynojirimycin (DNJNAc) were tested.

Specific inhibitors of β-N-acetythexosaminidases have received a great deal of attention mainly in order to be used for elucidating the role of β-N-acetythexosaminidases in biological processes but also as tools for development of therapeutic interventions. Specific use of such inhibitors as signal enhancing agents in enzyme based assays for fungal cells does not appear to have been considered previously.

So, in a first aspect the present invention relates a method for quantitative or qualitative determination of fungal cells in a sample potentially containing β-N-acetythexosaminidase 3.2.1.52 (NAHA) from non-fungal sources,, comprising contacting or mixing the sample with a substrate to produce a reaction mixture and subsequently assessing the numbers or concentration of the fungal cells in the sample as a function of measured conversion of the substrate in the reaction mixture after producing the reaction mixture, wherein
- the substrate can be converted by β-N-acetythexosaminidase3.2.1.52 (NAHA) produced by the fungal cells and by non-fungal NAHA, and
- a preferential inhibitor of the enzymatic conversion of the substrate by non-fungal NAHA is present in the reaction mixture;

wherein the preferential inhibitor is one that upon prolonged incubation with fungal cells inhibits the NAHA activity from fungal cells in a manner similar to the inhibition of non-fungal NAHA activity,
wherein the preferential inhibitor is a metal ion selected from Ag⁺, Hg²⁺, and Pb²⁺ or is 2-Acetamido-1,2-dideoxynojirimycin.

A second aspect of the invention relates to a method for determination of the relative amount of fungal cells in a location compared to at least one non-fungal cell in the same location, comprising the steps of
1) contacting or mixing a sample from the location with a first substrate to produce a first reaction mixture and subsequently determining the conversion rate of the first substrate after producing the first reaction mixture,
2) contacting or mixing a sample from the location with a second substrate to produce a second reaction mixture and subsequently determining the conversion rate of the second substrate after producing the second reaction mixture, and
3) determining the relative amount of the fungal cells by calculating the ratio between the conversion rate determined in step 1 and the conversion rate determined in step 2, wherein
   - the first and second substrates can be converted by both NAHA produced by the fungal cells and NAHA from non-fungal sources ,
   - a preferential inhibitor of the enzymatic conversion of the first substrate by NAHA from non-fungal sources is present in the first reaction
      mixture and a preferential inhibitor of the enzymatic conversion of the second substrate by NAHA from non-fungal sources is not present in the second reaction mixture;
      wherein the preferential inhibitor is one that upon prolonged incubation with fungal cells inhibits the NAHA activity from fungal cells in a manner similar to the inhibition of non-fungal NAHA activity,
      wherein the preferential inhibitor is a metal ion selected from Ag⁺, Hg²⁺, and Pb²⁺ or is 2-Acetamido-1,2-dideoxynojirimycin.

### LEGEND TO THE FIGURES

Fig. 1 shows the percentage distribution of the relative mould level in various samples.
Fig. 2 shows the percentage distribution of the relative mould level in samples from locations considered to have no mould problems (hatching with "/") and locations known to have mould problems (hatching with "\").

### DETAILED DISCLOSURE OF THE INVENTION

### Definitions

In the present description and claims, a "microorganism" is either a unicellular organism (e.g. a prokaryotic organism such as a bacterium or an archaea, or a unicellular eukaryotic organism such as a protist, a protozoan, an algae, a unicellular fungus, such as a yeast or fission yeast) or an organism which may be multicellular (e.g. filamentous fungi). Also included as microorganisms are those organisms that switch between unicellular forms and multicellular forms, i.e. slime molds, and certain fungi and algea. According to the present invention, it is of particular interest to apply the methods disclosed herein on fungi, in particular filamentous fungi/moulds.

A "substrate" is a substance which can undergo enzymatically catalysed conversion. Any such conversion may be of interest, as long as the substrate and at least one product of the enzymatic action on the substrate can be detected and distinguised. For example, the substrate may be non-detectable in a particular assay whereas the product is detectable or *vice versa;* or both the substrate and the product may be detectable but also distinguishable in the assay.

A "reaction mixture" is in the present context a mixture, which contains at least the sample, the substrate and optionally the preferential inhibitor. Other constituents may be present depending on the exact design of the assay in which the reaction mixture is an integral part.

A "preferential inhibitor" is a substance, which under the conditions specified for an assay (chemical and physical conditions as well as duration) provides for inhibition of conversion of the substrate by irrelevant cells to such a degree that the ratio between substrate converted by the at least one fungus of interest and substrate converted by irrelevant cells is increased at the point in time where the conversion is measured when compared to a situation where the inhibitor is not present. An inhibitor is therefore considered preferential for irrelevant cell NAHA even if it is in fact capable of inhibiting fungal NAHA to the same or a higher degree than the NAHA from irrelevant cells; for instance, if the time needed for the inhibitor to exert its full inhibitory effect on NAHA in the irrelevant cells is shorter than the time needed in the fungal cells of interest, the inhibitor will within a period of time after admixture with a sample comprising both fungal and irrelevant cells only effectively inhibit the NAHA in the irrelevant cells, and in this time window the conversion of substrate by NAHA will therefore be essentially limited to the conversion exerted by the fungal NAHA.

An "irrelevant cell" is a cell from any type of organism, which it is not of interest to determine when determining the microorganism of interest. Typically, the irrelevant cell will be that or those of an organism, which is present under normal circumstances in the sample material, but which due to the presence of equivalent enzymes as those sought detected in the fungal cells can give rise to false positive results, i.e. "noise".

In the present context, a "sample" is taken from any source material that might contain the microorganism of interest. Hence, the sample can for instance be an air sample, a dust sample, an earth sample a sample collected from a dry or wet surface, a sample from a liquid material such as water.

### Specific embodiments of the invention

As mentioned above, the first aspect of the invention relates to a method for quantitative or qualitative determination of fungal cells in a sample, comprising contacting or mixing the sample with a substrate to produce a reaction mixture and subsequently assessing the numbers or concentration of the fungal cells in the sample as a function of measured conversion of the substrate in the reaction mixture after producing the reaction mixture, wherein
- the substrate can be converted by β-N-acetyihexosaminidase 3.2.1.52 (NAHA) produced by the fungal cells and by NAHA produced by at least one non-fungal cell, and
- a preferential inhibitor of the enzymatic conversion of the substrate by NAHA produced by the at least one non-fungal cell is present in the reaction mixture;

wherein the preferential inhibitor is one that upon prolonged incubation with fungal cells inhibits the NAHA activity from fungal cells in a manner similar to the inhibition of non-fungal NAHA activity,
wherein the preferential inhibitor is a metal ion selected from Ag⁺, Hg²⁺, and Pb²⁺ or is 2-Acetamido-1,2-dideoxynojirimycin.

Typically, the fungal cells are fungal particles containing NAHA, such as filamentous fungal cell(s) in the form of hyphae or spores, hyphal fragments, or hyphal microfragments having sizes less than 1 µm, since the presently described method has been implemented as a variation of the fungus detection technology disclosed in WO 98/033934, but the practice of the invention is not limited to determination of filamentous fungi. The present invention has demonstrated that fungi are less sensitive to the inhibitory effect of both some non-specific and specific inhibitors of NAHA activity, while potential sources of false positive signals in samples suspected of containing fungi, notably such sources as pollen and house dust mites, are more sensitive. It is however more than likely that there also exists other "pairs" constituted by any microorganism of interest and a false positive signal generating microorganism, where the presently disclosed selective inhibition can be accomplished - one example could be algae vs. bacteria in a liquid sample.

Typically, the determination of the fungal cells is carried out by detecting the product of the conversion (*i*.*e*. detecting an increase in signal from the product) or detecting the substrate (*i*.*e*. detecting a decrease in signal from the substrate) - further, both substances may be detected. This means that the product of NAHA-driven conversion is detectable and/or wherein the unconverted substrate is detectable. It is preferred to only detect the product of the conversion due to sensitivity considerations - in most cases a surplus of substrate will be available in order to ensure a first order enzymatic reaction where only the concentration of the enzyme responsible for the degradation influences the conversion rate - and in such a case it is difficult to gauge a change in substrate concentration.

Any signal that can be conveniently detected can be used to measure the amount of conversion product or substrate. Typically the detectable product and/or the substrate is coloured, luminescent, fluorescent, chromogenic, enzymatically active, or a specific binding partner to a capture agent such as an antibody or a specific receptor. Due to the ease of measuring changes in fluorescence via photometric method, it is particularly preferred that the detectable substrate and/or product is fluorescent and that a change in fluorescence is gauged after addition of the substrate to the sample. In such cases, fluorescence is gauged intermittently or continuously, preferably over a period of about 30 minutes, for instance at one or several time points after addition of the substrate to the sample. Whether it is desirable to measure the fluorescence at one or several time points after addition of the substrate to the sample depends on the actual situation and the accuracy demanded by the test: if several time points are used, a curve over the change in fluorescence can be obtained and thereby the maximum conversion rate can be estimated (in particular the conversion rate when there is a large surplus of substrate so that the enzymes converting the substrate is operating at maximum speed and the curve is linear).

The period of about 30 minutes can be varied widely, and can e.g. be adjusted if the amount of fungal cells to be measured is known to be in a defined range or if the physical conditions of the determination introduce limitations on reaction speeds etc. A skilled person will readily be able to adapt a particular assay so that an optimized period of time is selected for the reaction between fungal cells and substrate - this is a simple task of calibrating a given assay. It is to be noted that in the event the inhibitor exhibits its differential inhibition due to differences in the time necessary for the inhibitor inhibit the NAHA 1) in the at least one fungus of interest and 2) in the false positive cells, this introduces an upper limit on the reaction time that can be allowed, which is set by the time it takes before the inhibitory effect on NAHA in both the at least one fungus of interest and in the irrelevant cells is at the same or similar level.

Typically, the inhibitor preferentially inhibits conversion of the substrate by the at least one non-fungal cell compared to the inhibition of the conversion by the fungal cells. As shown in the examples, using a standardized assay and carefully selected concentrations of inhibitor provides for a preferential inhibition of the NAHA activity in irrelevant cells, which is at least 10 times as high as the inhibition of the fungal enzyme activity. Expressed differently, the ratio (AMI₁ / AMI₀) / (AMN₁ / AMN₀) > 1, where AMI₁ and AMI₀ are the conversion rates (activities) of NAHA in the at least one fungus of interest with and without inhibitor added, respectively, and AMN₁ and AMN₀ are the conversion rates of NAHA in the irrelevant cells with and without inhibitor added, respectively. The ratio is typically >2, >3, >4, >5, >6, >6, >7, >8, >9, >10, >15, or even >20. An example is provided below in Example 2, where the ratio is about 7 at an inhibitor concentration of 24.5 µM inhibitor, and in Example 1, where the ratio was about 5.5 at an inhibitor concentration of 100 µM.

It is to be noted that the exact concentration of a given inhibitor used in the present invention has to be determined for a given practical application of the methods of the invention; see the examples. This is because the inhibitor has to be applied in a concentration, which is effective in providing inhibition of NAHA in at least the non-fungal cells. But, the effective concentrations of the different inhibitors useful in the present invention must be titrated against different concentrations of target NAHA (this can reflect various expected and realistic concentrations of fungi and competing cells in various types of sampling settings), and further, the effective concentrations of two different inhibitors vis-àvis the same concentration of NAHA will also be mutually independent. However, for most applications (i.e. determination of mould infestation in an indoor environment), such an effective concentration can be determined based on knowledge of known concentrations of non-fungel cells in a wide range of samples. Finally, the inhibitor will typically be used in a concentration in at least slight excess of the determined minimum effective concentration to ensure that unexpected higher concentrations of irrelevant cells are sufficiently inhibited.

The substrates used in the present invention (in particular for detection of filamentous fungi) are those that can be converted by β-N-acetythexosaminidase3.2.1.52 (NAHA). However, for other applications than those according to the invention it is contemplated that other characteristic enzymes can be used, depending on the exact organism it would be intended to determine. Also, NAHA is not the only enzyme which it would be possible to use in a equivalent method for fungus determination, thus giving rise to use of substrates specific for other fungal enzymes.

The detectable moiety in the substrate or the converted product is conveniently 4-methylumbelliferone or a fluorescently detectable derivative hereof when being converted by NAHA. Examples of such derivatives are 4-methylumbelliferone or a fluorescently detectable derivative hereof when being converted by NAHA, such as a methylumbelliferyl derivative selected from the group consisting of 4-methylumbelliferyl-β-N-acetyl-D-glucosaminide, 4-methylumbelliferyl-β-D-N,N',N"-triacetylchitotrioside, 5-bromo-6-chloro-3-indolyl-2-acetamido-2-deoxy-β-D-gluco-pyranoside, 5-bromo-4-chloro-3-indolyl-N-acetyl-β-D-glucosaminide, indolyl-2-acetamido-2-deoxy-β-D-gluco-pyranoside, 4-nitrophenyl-N-acetyl-β-D-glucosaminide, β-trifluoromethylumbelliferyl-N-acetyl-β-D-glucosaminide, N-methylum-indolyl-N-acetyl-β-D-glucosaminide, 5-iodo-3-indolyl-N-acetyl-β-D-glucosaminide, 4-methylumbelliferyl-β-D-N,N',N"-triacetylchitotriose, 4-methylumbelliferyl-β-D-N,N'-diacetylchitobioside, 4-methylumbelliferyl-7-(6-sulfo-2-acetamido-2-deoxy)-β-D-glucosaminide, 4-methylumbelliferyl α-D-fucoside; 4-methylumbelliferyl β-D-fucoside, 4-methylumbelliferyl α-D-glucoside, 4-methylumbelliferyl-7-(6-sulfo-2-acetamido-2-deoxy-β-D-glucopyronoside), 4-methylumbelliferyl-N-acetyl-α-D-glucosaminide, 4-methylumbelliferyl β-D-lactoside, 4-methylumbelliferyl-N-acetylgalactosaminide, 4-methylumbelliferyl β-D-mannopyranoside, 4-methylumbelliferyl α-D-mannopyranoside, 4-methylumbelliferyl β-D-xyloside, resorufin-N-acetyl-β-D-glucosaminide, 4-methylumbelliferyl-N-acetyl-α-D-glucosaminide, 9H-(1,3-dichloro-9,9-dimethylacridin-2-one-7-yl)N-acetyl-β-D-glucosa minide (DDAO), and an N-actyl-β-D-glucosaminide oligomer derivative of DDAO.

In particular preferred is a methylumbelliferyl derivative selected from the group consisting of 4-methylumbelliferyl-β-N-acetyl-D-glucosaminide and 4-methylumbelliferyl-β-D-N,N',N"-triacetylchitotrioside.

As shown in the examples, the inhibitor can be a specific as well as an unspecific NAHA inhibitor. Unspecific inhibitors are metal ions selected from Ag⁺, Hg²⁺, and Pb^{2+.} An example of a specific NAHA inhibitor is 2-Acetamido-1,2-dideoxynojirimycin.

As mentioned in the examples, one of the possible reasons for the preferential inhibition exploited in the present invention is that the fungal cell to be tested does not permit the inhibitor to gain access to NAHA at the same speed as do the irrelevant cell(s) - for this reason and as a precaution, it is preferred that the inhibitor is not brought into contact with the sample prior to the contact between the substrate and the sample. Hence the substrate and the inhibitor may be mixed prior to contact with the sample, or they may be added to the sample at substantially the same time.

However, it is according to the present invention possible to add the inhibitor to the sample prior to the addition of the substrate, as long as this will not substantially have a negative impact on the preferential inhibition of NAHA activity that is aimed at in the assay. In other words, the preferential inhibitor may be
- mixed or contacted with the substrate prior producing the reaction mixture, or
- added simultaneously with the substrate to the sample when producing the reaction mixture, or
- added to the sample prior to producing the reaction mixture.

In the latter of these 3 cases the inhibitor is preferably added to the sample at most 2 hours prior to producing the reaction mixture, such as the most 115 minutes, at most 100 minutes, at most 105 minutes, at most 100 minutes, at most 95 minutes, at most 90 minutes, at most 85 minutes, at most 80 minutes, at most 75 minutes, at most 70 minutes, at most 65 minutes, at most 60 minutes, at most 55 minutes, at most 50 minutes, at most 45 minutes, at most 40 minutes, at most 35 minutes, at most 30 minutes, at most 25 minutes, at most 20 minutes, at most 15 minutes, at most 10 minutes, and at most 5 minutes prior to producing the reaction mixture.

In the practice of the first aspect, the fungal cell level is typically determined based on a series of calibration measurements performed on standards comprising known concentrations of the fungal cells in question. Alternatively, the calibration is based on empirical data obtained from environments that are known to exhibit various levels of contamination with the at least one fungus of interest.

As shown in the examples, the use of very different inhibitors of NAHA (both specific, and more general enzyme inhibitors) have proven successful in the practice of the first aspect of the invention. Further, as also discussed herein, prolonged incubation of these inhibitors with fungal cells has the effect that NAHA activity from fungal cells is inhibited in a manner similar to the inhibition of non-fungal NAHA.

This has led the inventor to conclude that the principle of enhancing specificity of a microbial assay by using inhibitors in a manner analogous to the one described for NAHA inhibition can be applied in a more general way. At least, the data presented herein provide strong evidence that *any* determination of fungal cell number/concentration, which relies on an assay of the activity of fungal enzymes that have counterparts in other cells that are irrelevant for the determination, can be rendered more specific by incorporating inhibitors of the enzyme activity during the course of the assay. This is so because the experimental data presented strongly suggest that any inhibitor of enzyme activity will be "delayed" in its access to the enzyme that it interacts with in fungal cells, when comparing to irrelevant cells in the test sample. In other words, the present invention appears to demonstrate that many substances - such as the various inhibitors tested herein - for some unknown reason have easier access to their target enzyme in non-fungal cells compared to their access in the fungal cells, whereas the specific enzyme inhibited is of minor relevance.

This thus opens for a a method for quantitative or qualitative determination of at least one fungus of interest in a sample, comprising contacting or mixing the sample with an enzyme substrate to produce a reaction mixture and subsequently assessing the numbers or concentration of the at least one fungus of interest in the sample as a function of measured conversion of the substrate in the reaction mixture after producing the reaction mixture, wherein
- the substrate can be converted by an enzyme produced by the at least one fungus of interest and by a competing enzyme produced by at least one irrelevant cell, and
- a preferential inhibitor of the enzymatic conversion of the substrate by the enzyme and the competing enzyme is present in the reaction mixture.

Hence, all embodiments not directly linked to NAHA and substrates thereof described above for the first aspect with respect to modes of carrying out the method are applicable for this more versatile method. The skilled person will be able to select suitable substrates for a selected enzyme that is useful for fungal cell selection and also able to select specific inhibitors of such an enzyme (keeping in mind that the unspecific inhibitors such as silver ions and the other non-specific inhibitors will have a broad applicability in any such assay).

### Second aspect of the invention - relative determination of microorganisms

As evident from the above, the method of the first aspect of the invention identifies - in a preferential manner - those fungi (typically filamentous fungi such as moulds) that - at least for a period - are less susceptible to the inhibiting effect of the NAHA inhibitor that is present in the reaction mixture. As also mentioned, if the inhibitor is not present in the reaction mixture, the determination of the fungal cells via substrate conversion methods, such as those of the prior art discussed above, can lead to false positive results due to conversion of the substrate by NAHA present in cells that are not relevant for the determination in question.

It has now been found by the inventor that if the 2 measurement types are run in parallel on samples from the same location (such as the same room or building), the ratio between the two measurements can provide a clear indication of the relative amount of the microorganisms in the total amount of cells measured and also provide a qualitative indication of the presence of a possible sources thereof.

In practice, the locations that are examined with the method of the first aspect of the invention (and with prior art methods), may be subject to highly varied conditions in terms of cleaning and ventilation: The presence of fungi such as allergenic moulds is determined by collecting samples from the location suspected of being infested. However, if such a location is ventilated effectively several times per day or has recently been subjected to extensive cleaning, the presence of mould-derived material in samples obtained will be low and a measurement performed according to the first aspect of the invention or according to the prior art methods could provide the false impression that the location is not infested with moulds. However, by assessing the relative amount of moulds within the total amount of cells capable of converting the NAHA substrate used in the determination, it becomes possible to identify the presence of an infestation even in locations where the majority of measurable cells and moulds have been removed, for instance due to the ventilation or cleaning.

As is evident from example 3, there is generally a clear distinction between the relative amount of measurable moulds in samples from locations regarded as being free of mould infestation and locations where moulds are recognized as a problem. Generally speaking, locations that provide for measurements that show a relative mould content of less than about 35% will normally not be regarded as being infested with moulds unless the absolute amount of moulds is beyond the threshold regarded as acceptable. Thus, with the present invention it is now possible to provide a much more detailed analysis of the status of mould infestation in a location:
1) If the total concentration of moulds is above the acceptable threshold as measured according to the first aspect of the invention, it can be concluded that a mould infestation is present and has to be dealt with.
2) If the total concentration of moulds is below the acceptable threshold level, a measurement of the relative amount of moulds according to the 2^{nd} aspect of the invention will be able to provide information about a possible mould infestation problem - e.g. if the relative amount of mould activity turns out to be ≥45%, then even a modest or low measurement according to the first aspect of the invention will reflect a local source of moulds that has not been detectable because e.g. of frequent and extensive ventilation or frequent and extensive cleaning. In other words, the method of the 2^{nd} aspect of the invention enables the detection of hidden sources of moulds that might be masked by other factors such as ventilation and cleaning. For measurements that provide values in the range between ≥36% and <45%, will normally have to be investigated further, since no definite conclusion concerning mould infestation can be made.

A third information that can be provided by the method of the 2^{nd} aspect appears in the event that the non-inhibited reaction mixture provides for a very high readout, whereas the inhibited reaction mixture does not. This provides the information that the sample location is not infested with moulds, but that the amount of "irrelevant" cells is high. This can e.g. trigger a recommendation that the sample location be made the subject of thorough cleaning or disinfection.

Both the measurement of the fungal cells and the total (measurable) biomass in a sample can conveniently be based on the substrate conversion rates that have been calibrated against standard samples, so that a conversion rate from each type of measurement is translated into a number that indicates the microorganism concentration and total cell concentration, respectively. As mentioned above, an alternative to calibration against standards is to perform measurements in locations having relatively well-defined levels of the at least one fungus of interest and use these initial measurements as calibration values.

Thus, as mentioned above, the 2^{nd} aspect of the invention relates to a method for determination of the relative amount of fungal cells in a location compared to at least one non-fungal cell in the same location, comprising the steps of
1) contacting or mixing a sample from the location with a first substrate to produce a first reaction mixture and subsequently determining the conversion rate of the first substrate after producing the first reaction mixture,
2) contacting or mixing a sample from the location with a second substrate to produce a second reaction mixture and subsequently determining the conversion rate of the second substrate after producing the second reaction mixture, and
3) determining the relative amount of the at least one fungus of interest by calculating the ratio between the conversion rate determined in step 1 and the conversion rate determined in step 2, wherein
   - the first and second substrates can be converted by both NAHA produced by the fungal cells and NAHA from non-fungal sources,
   - a preferential inhibitor of the enzymatic conversion of the first substrate by NAHA from non-fungal sources is present in the first reaction mixture and a preferential inhibitor of the enzymatic conversion of the second substrate by NAHA from non-fungal sources is not present in the second reaction mixture;

wherein the preferential inhibitor is one that upon prolonged incubation with fungal cells inhibits the NAHA activity from fungal cells in a manner similar to the inhibition of non-fungal NAHA activity,
wherein the preferential inhibitor is a metal ion selected from Ag⁺, Hg²⁺, and Pb²⁺ or is 2-Acetamido-1,2-dideoxynojirimycin.

The two samples tested are conveniently aliquots derived from the same original sample taken at the location in order to ensure that the samples reflect the same proportion of fungal cells and the irrelevant cells.

The "location" it typically an indoor environment and can be a single room but also an entire house where samples such as air samples or surface samples are collected at multiple sampling sites.

In general, step 1 of the 2^{nd} aspect of the invention is carried out in the same manner as when carrying out the method of the first aspect of the invention. In other words, all considerations relating to the sample type, the microorganism to be determined, the choice of substrate, the measurement details etc. apply *mutatis mutandis* to step 1 in the first aspect and to the extent that the means and measures used to carry out the first aspect of the invention are not related to the use of the inhibitor, the details pertaining to the embodiments of the first aspect of the invention also apply *mutatis mutandis* to 2^{nd} step in the method of the 2^{nd} aspect.

In the 2^{nd} aspect of the invention, it is therefore convenient but not necessary that the 1^{st} and 2^{nd} substrates are identical: Since the conversion rate of each substrate is typically translated into an indication of cell concentration via a calibration against relevant standard samples, it is in practice of the invention not an absolute necessity that the two substrates are the same in order to obtain a meaningful and useful result, but in practice it is more convenient to use the same reagents throughout the testing.

As with the first aspect of the invention, the fungal cells are in important embodiments selected from the group of filamentous fungi (moulds) in the form of hyphae or spores.

As is the case in the first aspect of the invention, it is a matter of choice whether the product(s) of conversion by NAHA is detectable and/or wherein the unconverted substrate(s) is/are detectable, meaning that conversion can be measured by gauging the increase in the amount of the product of the conversion(s) and/or the decrease in the amount of the substrate(s). It is normally most convenient to gauge the increase in the amount of the product of the conversion because the relative changes in signal - especially initially - are much higher than if measuring the decrease in substrate amount.

As is also the case in the 1^{st} aspect the detectable product(s) and/or the detectable substrate(s) can be coloured, luminescent, fluorescent, chromogenic, enzymatically active, or a specific binding partner to a capture agent. It is preferred that the detectable substrate and/or product is/are fluorescent and that a change in fluorescence is gauged after addition of the substrate to the sample. Fluorescence can be gauged intermittently or continuously, preferably over a period of about 30 minutes - whether it is desirable to measure the fluorescence at one or several time points after addition of the substrate to the sample depends on the actual situation and the accuracy demanded by the test: if several time points are used, a curve over the change in fluorescence can be obtained and thereby the maximum conversion rate can be estimated (i.e. the conversion rate when there is a large surplus of substrate so that the NAHA converting the substrate is operating at maximum speed and the curve is linear).

Also, the preferential inhibitor preferentially inhibits conversion of the first substrate by the at least one non-fungal cell compared to the inhibition of the conversion by the fungal cells.

Particular preferred substrates are those that release 4-methylumbelliferone or a fluorescently detectable derivative hereof when being converted by NAHA. Examples of such derivatives are selected from the group consisting of 4-methylumbelliferone or a fluorescently detectable derivative hereof when being converted by NAHA, such as a methylumbelliferyl derivative selected from the group consisting of 4-methylumbelliferyl-β-N-acetyl-D-glucosaminide, 4-methylumbelliferyl-β-D-N,N',N"-triacetylchitotrioside, 5-bromo-6-chloro-3-indolyl-2-acetamido-2-deoxy-β-D-gluco-pyranoside, 5-bromo-4-chloro-3-indolyl-N-acetyl-β-D-glucosaminide, indolyl-2-acetamido-2-deoxy-β-D-gluco-pyranoside, 4-nitrophenyl-N-acetyl-β-D-glucosaminide, β-trifluoromethylumbelliferyl-N-acetyl-β-D-glucosaminide, N-methylum-indolyl-N-acetyl-β-D-glucosaminide, 5-iodo-3-indolyl-N-acetyl-β-D-glucosaminide, 4-methylumbelliferyl-β-D-N,N',N"-triacetylchitotriose, 4-methylumbelliferyl-β-D-N,N'-diacetylchitobioside, 4-methylumbelliferyl-7-(6-sulfo-2-acetamido-2-deoxy)-β-D-glucosaminide, 4-methylumbelliferyl α-D-fucoside; 4-methylumbelliferyl β-D-fucoside, 4-methylumbelliferyl o-D-glucoside, 4-methylumbelliferyl-7-(6-sulfo-2-acetamido-2-deoxy-β-D-glucopyronoside), 4-methylumbelliferyl-N-acetyl-α-D-glucosaminide, 4-methylumbelliferyl β-D-lactoside, 4-methylumbelliferyl-N-acetylgalactosaminide, 4-methylumbelliferyl β-D-mannopyranoside, 4-methylumbelliferyl α-D-mannopyranoside, 4-methylumbelliferyl β-D-xyloside, resorufin-N-acetyl-β-D-glucosaminide, 4-methylumbelliferyl-N-acetyl-α-D-glucosaminide, 9H-(1,3-dichloro-9,9-dimethylacridin-2-one-7-yl)N-acetyl-β-D-glucosa minide (DDAO), and an N-actyl-β-D-glucosaminide oligomer derivative of DDAO.

In particular preferred is a methylumbelliferyl derivative selected from the group consisting of 4-methylumbelliferyl-β-N-acetyl-D-glucosaminide and 4-methylumbelliferyl-β-D-N,N',N"-triacetylchitotrioside.

As in the first aspect, the inhibitor can be both a specific and an unspecific NAHA inhibitor. Unspecific inhibitors are metal ions selected from Ag⁺, Hg²⁺, and Pb²⁺. The specific NAHA inhibitor is 2-Acetamido-1,2-dideoxynojirimycin.

As mentioned in the examples, one of the possible reasons for the preferential inhibition exploited in the present invention is that the fungal cells to be detected do not permit the inhibitor to gain access to the NAHA at the same speed as do the irrelevant cell(s) - for this reason and as a precaution, it is preferred that the inhibitor is not brought into contact with the sample prior to the contact between the substrate and the sample. Hence the substrate and the inhibitor may be mixed prior to contact with the sample, or they may be added to the sample at substantially the same time.

However, it is according to the 2^{nd} aspect of the invention possible to add the inhibitor to the sample prior to the addition of the substrate in step 1, as long as this will not substantially have a negative impact on the preferential inhibition of NAHA activity that is aimed at in the assay. In other words, the preferential inhibitor may be
- mixed or contacted with the substrate prior producing the reaction mixture, or
- added simultaneously with the substrate to the sample when producing the reaction mixture, or
- added to the sample prior to producing the reaction mixture.

In the latter of these 3 cases the inhibitor is preferably added to the sample at most 2 hours prior to producing the reaction mixture, such as the most 115 minutes, at most 100 minutes, at most 105 minutes, at most 100 minutes, at most 95 minutes, at most 90 minutes, at most 85 minutes, at most 80 minutes, at most 75 minutes, at most 70 minutes, at most 65 minutes, at most 60 minutes, at most 55 minutes, at most 50 minutes, at most 45 minutes, at most 40 minutes, at most 35 minutes, at most 30 minutes, at most 25 minutes, at most 20 minutes, at most 15 minutes, at most 10 minutes, and at most 5 minutes prior to producing the reaction mixture.

In line with the first aspect of the invention, the second aspect also enable a broader version, which is not part of the invention, due to the finding that the preferential inhibition utilised is due to a characteristic of the fungal cells that apparently slow down the inhibitor's access to its enzyme target - cf. the explanation above in the last sections of the description of the 1^{st} aspect. This broader version of the 2^{nd} aspect can be described as a method for determination of the relative amount of at least one fungus of interest in a location compared to at least one irrelevant cell in the same location, comprising the steps of
1) contacting or mixing a sample from the location with a first enzyme substrate to produce a first reaction mixture and subsequently determining the conversion rate of the first enzyme substrate after producing the first reaction mixture,
2) contacting or mixing a sample from the location with a second enzyme substrate (which may be identical to the first substrate) to produce a second reaction mixture and subsequently determining the conversion rate of the second enzyme substrate after producing the second reaction mixture, and
3) determining the relative amount of the at least one fungus of interest by calculating the ratio between the conversion rate determined in step 1 and the conversion rate determined in step 2, wherein
   - the first and second substrates can be converted by an enzyme produced by the at least one fungus of interest and an enzyme produced by the at least one irrelevant cell,
   - a preferential inhibitor of the enzymatic conversion of the first substrate by the enyzmes produced by the at least one fungus and by the at least one irrelevant cell is present in the first reaction mixture and such a preferential inhibitor is not present in the second reaction mixture.

Hence, all embodiments not directly linked to NAHA and substrates thereof described above for the 2^{nd} aspect of the invention with respect to modes of carrying out the method are applicable for this more versatile method. The skilled person will be able to select suitable substrates for a selected enzyme that is useful for fungal cell selection and also able to select specific inhibitors of such an enzyme (keeping in mind that the unspecific inhibitors such as silver ions and the other non-specific inhibitors will have a broad applicability in any such assay).

### EXAMPLE 1

### Ag⁺ as an inhibitor of NAHA

In order to determine NAHA activity in different samples, the technology in WO 98/033934 was employed: A sample containing a dried or freeze dried organism is added to a buffer solution containing 4-Methylumbelliferyl N-acetyl-β-D-glucosaminide in a concentration of 20 µM. The reaction-time is varied depending on the ambient temperature and according to a reaction-time/temperature data sheet previously developed for fungal NAHA activity at various temperatures, and is 30 minutes at 23°C. The resulting fluorescence formed during the reaction time is monitored in a calibrated fluorometer.

In a pilot experiment Ag⁺ in a concentration of 10 µM exhibited an almost total inhibition of NAHA activity in grass pollen and in the mite Lepidoglyphus. On the other hand, Ag⁺ exhibited no significant effect on the NAHA activity in three tested fungi (*Alternaria* spp., *Acremonium* spp. and *Cladosporium* spp.).

Hg⁺ (10 µM) had a similar effect with high inhibition of non-fungal and less inhibition (but more than Ag⁺) of fungal NAHA activity. Cu²⁺ in concentrations of 500 µM on the other hand did not exithibit any or exhibited only very slight inhibiting effect on NAHA activity from all sources.

From these results and due to the higher toxicity of Hg⁺ it was decided to only focus on the effect of Ag⁺ in the further work. Experiments were conducted where varying concentrations of AgNO₃ was added to an analysis substrate. Furthermore, the experiments where performed on dried or freeze dried materials where both intact and fragmented cells are to be expected to be present. Table 1 shows the effect of Ag⁺ in increasing concentrations on NAHA activity in non-fungal biomass sources and table 2 shows the effect on NAHA from fungal biomass sources.

The results evidence that the level of enzyme activity is dependent on the concentration of Ag⁺ but even at 400 µM, a high percentage of the fungal NAHA activity is retained while the non-fungal sources only retains an average of 13% of the control. On average, an Ag⁺ concentration of 100 µM in the assay substrate lead to a reduction of 82% of the non-fungal NAHA activity and from zero to very low reduction of the fungal NAHA activity. Hence, use of Ag⁺ provided a significant reduction of false positive signals and thereby results in increased specificity of the enzyme activity measurements. In turn this increase in specificity (which is accompanied by a minute loss of sensitivity in detection of fungal NAHA activity) provides for an unexpected increas in the assay's accuracy.

**Table 1**

| The effect of varying concentrations of AgNO₃ concentrations on the NAHA activity from various sources. The enzyme assays were run for 30 min. The control (the assay without Ag⁺) were used as 100% activity. | | | | | | | | | |
|---|---|---|---|---|---|---|---|---|---|
| **Ag+ (µM)** | Grahamflour | Ambrosia psilostachya (Rag weed pollen) | Cat dander | Human skin cells | Mugworth pollen | Lepidoglyphus destructor (Storage mite) | Grass pollen | Dermatophagoides pteronyssinus (Dust mite) | Birch pollen |
| **0** | 100% | 100% | 100% | 100% | 100% | 100% | 100% | 100% | 100% |
| **10** | 35% | 69% | 96% | 67% | 46% | 3% | 41% | 80% | 91% |
| **25** | 33% | 55% | 69% | 65% | 23% | 2% | 22% | 57% | 74% |
| **100** | 25% | 19% | 6% | 63% | 9% | 1% | 6% | 13% | 18% |
| **400** | 19% | 17% | 3% | 46% | 8% | 0% | 8% | 6% | 11% |

**Table 2**

| The effect of varying concentrations of AgNO₃ concentrations on the NAHA activity from different fungi. | | | | | | | | | |
|---|---|---|---|---|---|---|---|---|---|
| **Ag+ (µM)** | *Aspergillus fumigatus* freeze dried | *Aspergillus fumigatus fresh from cultivation* | Acremonium spp. | Penicillium crysogenum | Cladosporium sphaerospermum | Fusarium culmorum | Mucor hiemalis | Stachybotrys chartarum | Aureobasidium pullulans |
| **0** | 100% | 100% | 100% | 100% | 100% | 100% | 100% | 100% | 100% |
| **10** | 104% | 91% | 175% | 98% | 212% | 107% | 88% | 97% | 89% |
| **25** | 108% | 103% | 143% | 106% | 154% | 107% | 63% | 91% | 91% |
| **100** | 98% | 95% | 159% | 87% | 137% | 110% | 114% | 83% | 96% |
| **400** | 91% | 87% | 132% | 65% | 167% | 102% | 57% | 93% | 76% |

### EXAMPLE 2

### 2-Acetamido-1,2-dideoxynojirimycin (DNJNAc) as inhibitor

Experiments analogous to those in example 1 were carried out, but instead of using non-specific inhibitors, the effect of different concentrations of DNJNAc on the NAHA activity in various biomass containing samples was investigated. Table 3 shows the effects observed in samples of non-fungal biomass, and Tables 4a and 4b shows the effects observed in samples with fungal biomass.p

The results shown in these tables evidence that the level of enzyme activity is dependent on the concentration of DNJNAc. At the highest concentration tested (196 µM), an average of 60% of the fungal NAHA activity was retained while the non-fungal sources only retained an average of 5% of the control. With a DNJNAc concentration of 24.5 µM in the assay substrate, an average of 11% of the non fungal activity was retained while the average of the fungal NAHA was 83%.

**Table3**

| The effect of various concentrations of DNJNAc in the assay substrate, on the NAHA activity from non-fungal sources. | | | | | | | | | |
|---|---|---|---|---|---|---|---|---|---|
| **DNJNAc (µM)** | Graham flour | *Ambrosia psilostachya* (Rag weed pollen) | Cat dander | Human skin cells | Mugworth pollen | *Lepidoglyphus destructor* (Storage mite) | Grass pollen | *Dermatophagoides oteronyssinus* (House dust mite) | Birch pollen |
| **0** | 100% | 100% | 100% | 100% | 100% | 100% | 100% | 100% | 100% |
| **24.5** | 14% | 8% | 7% | 3% | 8% | 3% | 41% | 5% | 10% |
| **49** | 9% | 6% | 5% | 1% | 6% | 2% | 37% | 2% | 9% |
| **98** | 6% | 5% | 3% | 1% | 5% | 1% | 30% | 1% | 8% |
| **196** | 6% | 4% | 2% | 1% | 4% | 0% | 20% | 1% | 9% |

**Table 4a**

| The effect of various conc entrations of DNJNAc in assay subtrate, on the NAHA activity from rungal sources. | | | | | | | | |
|---|---|---|---|---|---|---|---|---|
| **DNJNAc (µM)** | *Mucor hiemalis* | *Rhizopus stolonifer* | *Fusarium culmorum* | *Chaetomium qlobosum* | *Aureobasidium pullulans* | *Aspergillus versicolor* | *Alternaria tenuissima* | *Trichoderma harzianum* |
| **0** | 100% | 100% | 100% | 100% | 100% | 100% | 100% | 100% |
| **24.5** | 58% | 46% | 85% | 88% | 56% | 92% | 72% | 120% |
| **49** | 37% | 32% | 82% | 91% | 67% | 79% | 79% | 93% |
| **98** | 45% | 23% | 98% | 92% | 50% | 71% | 58% | 92% |
| **196** | 25% | 18% | 95% | 81% | 27% | 63% | 48% | 83% |

**Table 4b**

| The effect of various concentrations of DNJNAc in the assay substrate, on the NAHA activity from fungal sources. | | | | | | | |
|---|---|---|---|---|---|---|---|
| **DNJNAc (µM)** | *Acremonium* sp. | *Stachybotrys chartarum* | *Chladosporium sphaerosperm* | *Penicillium commune* | *Aspergillus fumigatus* | *Penicillium chrysogenum* | *Aspergillus fumigatus* (Freeze dried) |
| **0** | 100% | 100% | 100% | 100% | 100% | 100% | 100% |
| **24.5** | 99% | 90% | 43% | 76% | 122% | 89% | 114% |
| **49** | 92% | 104% | 32% | 62% | 110% | 86% | 105% |
| **98** | 80% | 97% | 49% | 45% | 89% | 74% | 104% |
| **196** | 76% | 85% | 21% | 22% | 88% | 76% | 96% |

### DISCUSSION OF RESULTS IN EXAMPLES 1 AND 2

The concentration of NAHA inhibitors is important in order to obtain selectivity of inhibition: If using too high a concentration, both fungal and non-fungal NAHA activity will be inhibited. But the examples demonstrate that there exists a window of inhibitor concentrations where fungal NAHA activity is only slightly inhibited while non-fungal NAHA activity is almost completely inhibited; in other words, at certain concentrations of inhibitor, non-fungal NAHA activity is preferentially suppressed.

It is surprising that this selective effect can be obtained with both non-specific enzyme inhibitors such as silver and mercury ions and with more specific types of NAHA inhibitors such as DNJNAc. One would expect that silver/mercury would work differently than a glyconbased substrate analogue such as DNJNAc.

The experiments have not revealed the mechanism(s) involved in the preferential inhibition. The experiments were performed with whole cell/organism cultures rather than purified enzyme, which is normally used in experiments on specific inhibition. It is plausible, though, that the surprising non-specific inhibition effect observed for Ag⁺ and Hg⁺ could be due to the inhibitors being delayed in physically contacting the NAHA enzyme in the fungi when compared to other types of biomass. This is supported by the finding that addition of Ag⁺ to the fungal culture prior to the enzyme assay leads to a complete inhibition of the fungal NAHA activity in line with the inhibition of other types of biomass.

### EXAMPLE 3

### Determination of relative mould presence in samples

By comparing the NAHA activity with and without a NAHA inhibitor such as DNJNAc in parallel samples (air or surface dust) it is possible to provide answers to 3 questions; 1) What is the quantitative amount/level of mould (measurement in the presence of inhibitor) in the sample, 2) what is the quantitative amount/level of (micro)organisms (measurement in the absence of inhibitor), and 3) what is the relative mould level by calculating the proportion that the mould level constitutes of the general microorganism level?

Samples from buildings with and without mould problems were obtained. Aggressive air sampling was used according to the following protocol for aggressive air sampling.

### Aggressive air sampling protocol

| | |
|---|---|
| Sample volume: | 150-300 l |
| Flow rate: | 10-20 l/min. 20 l/min is the ideal flow rate, 10 is the minimum flow rate (note the flowrate and use for calculating sample volume). Minimum flowrate for Quality Control of cleanliness is 15 l/min (225 l sampling volume). |
| Sampling time: | 15 min. (always). |
| Sampling height: | Approx. 1.5 m. |
| Filter: | Use only MM-air filter (MCE filter pore size, 0.8 µm). |
| Filter orientation: | The entire lid is removed and is placed with opening facing upwards. |
| Safety: | Respiratory protection should be used by everyone present in the room. |
| Room size: | One sample in a room up to 60 m2. In bigger rooms take several samples. |

All windows should be closed for at least 6 hours before sampling. Note if there is mechanical ventilation, dehumidifiers, air purifiers or the like running.
1. Set up pump and tripod.
2. Give the filter an ID and put it on the tube.
3. Set one timer on two min. and one on 15 min.
4. Put on the respiratory protection.
5. Now *blow* on any surface with a distance of approx. 2 meter with a Makita blower (speed setting 3). Start with the ceiling, then the walls ending with furniture and the floor. Do not blow from a short distance to release dust from reservoirs that are almost newer cleaned (e.g., between the lamella of a radiator). Use the blower approx. 2 min in a room of 20 m² and extra-/interpolate from that to smaller or bigger rooms.
6. When finished with the blowing, start the stopwatch (2 min.)
7. When the stopwatch signals, remove the lid of the filter (not just the blue stopper), and start the air pump and the timer (15 min.).
8. Adjust the flow rate 20 l / min.
9. When the timer signals, stop the pump and put the lid back on the filter.

The 2 min. pause between blasting and starting the air pump is introduced to avoid any influence from large dust balls that have been blown up. If several samples are taken in succession, or if there are many particles in the air, the pump can become very warm which can affect the flow rate. Therefore, check regularly that the flow rate is correct during sampling. Be aware that if the pump gets very warm, they often have a mechanism that shuts them off. Deviations from this protocol can affect sampling efficiency which means that the results categories cannot be applied.

The air samples must be analyzed within 1 week from sampling date when stored at room temperature. It is always recommended to analyze samples as soon as possible after sampling.

### Experimental

A number of parallel twin samples were taken as described above and subsequently analyzed for NAHA activity; for each set of twin samples, one sample was analysed with and the other without DNJNAc as an additive in the substrate solution.

The samples analysed in the absence of DNJNAc and other inhibitors represent the NAHA activity from all sources of indoor cellular materials that exhibit NAHA activity; fungi, dust mites, pollen, dog-, and cat dander and others as well as human skin scales. Samples analysed in the presence of DNJNAc represent fungi as demonstrated in example 2. By comparing the results from the two parallel samples, the relative amount of mould or the relative mould level/index is calculated, as the NAHA activity with DNJNAc, divided by the NAHA activity without DNJNAc. So the two parallel samples provide three pieces of information; 1) the level of mould, 2) the level of general cellular material, and 3) the relative level of mould to general cellular material.

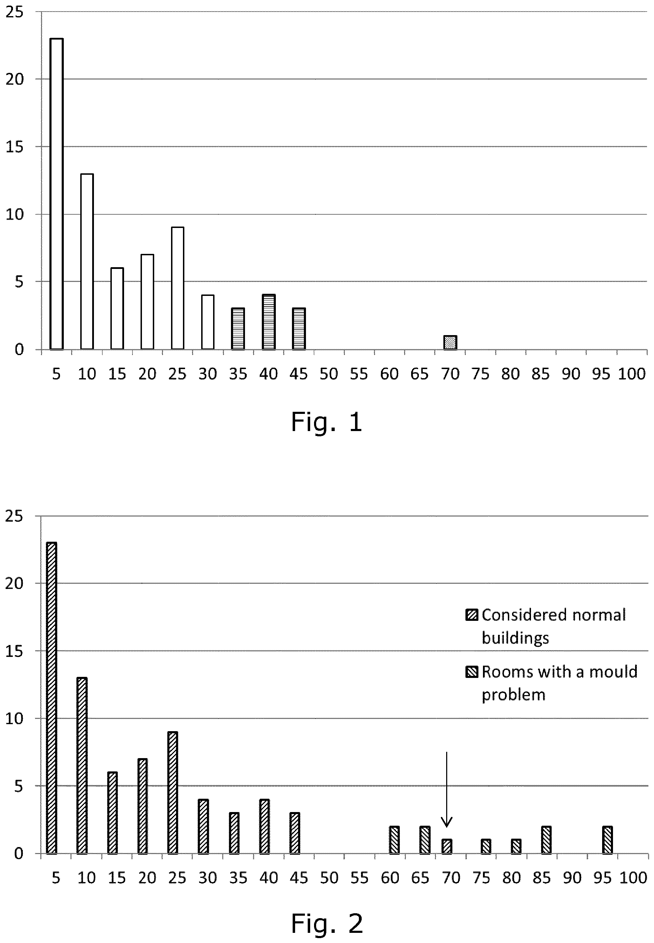

Fig. 1 shows the distribution of the relative mould levels measured in samples: 84.4% of the samples had a relative mould level of 30% or less (white bars). 13% of the samples had a relative mould level above 30% and below or equal to 45%, (horizontally hatched bars) and 2.6% had a relative mould level above 45% (cross-hatched bar).

Table 4 shows the NAHA activity with and without DNJNAc added to enzyme reaction in air samples from different rooms in 18 buildings considered being non-problem buildings (in relation to mould problems and water damage). The relative mould index is calculated, as the NAHA activity with DNJNAc, divided by the NAHA activity without DNJNAc.

**Table 4**

| | | NAHA activity | | Relative | | | | NAHA activity | | Relative |
|---|---|---|---|---|---|---|---|---|---|---|
| | | Without | With | mould | | | | Without | With | mould |
| Building | | DNJNAc | DNJNAc | level % | | Building | | DNJNAc | DNJNAc | level % |
| 1 | Bedroom 1 | 578 | 22 | 4 | | 11 | Bathroom 1 | 311 | 62 | 20 |
| 1 | Bedroom 2 | 711 | 36 | 5 | | 11 | Lounge | 142 | 62 | 44 |
| 1 | Bedroom 3 | 249 | 9 | 4 | | 11 | Dressing room | 293 | 31 | 11 |
| 1 | Living room | 156 | 4 | 3 | | 11 | Bedroom 1 | 502 | 11 | 2 |
| 1 | Dining room | 315 | 22 | 7 | | 13 | Bedroom 1 | 1356 | 102 | 8 |
| 1 | Play room | 369 | 138 | 37 | | 13 | Bathroom | 502 | 0 | 0 |
| 1 | Bathroom | 147 | 0 | 0 | | 13 | Kitchen | 129 | 44 | 34 |
| 2 | Sun room | 111 | 18 | 16 | | 13 | Dining room | 307 | 98 | 32 |
| 2 | Lounge | 231 | 62 | 27 | | 14 | Bedroom | 955 | 58 | 6 |
| 2 | Bedroom 1 | 578 | 22 | 4 | | 14 | Lounge/Diner | 710 | 18 | 3 |
| 3 | Bedroom 2 | 711 | 36 | 5 | | 15 | Office | 293 | 67 | 23 |
| 3 | Bedroom 3 | 249 | 9 | 4 | | 15 | Kitchen | 490 | 53 | 11 |
| 3 | Living room | 156 | 4 | 3 | | 15 | Bedroom | 257 | 17 | 7 |
| 3 | Dining room | 315 | 22 | 7 | | 15 | Lounge | 237 | 13 | 5 |
| 3 | Play room | 369 | 138 | 37 | | 15 | Bedroom | 357 | 80 | 22 |
| 3 | Bathroom | 147 | 0 | 0 | | 16 | Office | 764 | 27 | 4 |
| 3 | Office | 302 | 22 | 7 | | 16 | Bedroom | 316 | 18 | 6 |
| 4 | Lounge | 191 | 9 | 5 | | 16 | Bedroom2 | 316 | 4 | 1 |
| 4 | Kitchen | 258 | 44 | 17 | | 16 | Lounge | 142 | 0 | 0 |
| 4 | Bathroom | 378 | 22 | 6 | | 16 | Kitchen/Diner | 116 | 0 | 0 |
| 5 | Bedroom | 93 | 22 | 24 | | 16 | Bathroom | 316 | 40 | 13 |
| 5 | Bathroom | 378 | 22 | 6 | | 16 | WC | 1053 | 67 | 6 |
| 6 | Bedroom | 93 | 22 | 24 | | 16 | Hallway | 111 | 40 | 36 |
| 6 | Sun room | 111 | 18 | 16 | | 16 | En-suite | 978 | 71 | 7 |
| 7 | Lounge | 231 | 62 | 27 | | 17 | Hall | 182 | 0 | 0 |
| 7 | Kitchen 1 | 249 | 0 | 0 | | 17 | Livingroom | 249 | 93 | 37 |
| 7 | Bedroom 1 | 289 | 11 | 4 | | 17 | Livingroom | 186 | 124 | 67 |
| 8 | L1 | 573 | 84 | 15 | | 17 | Bedroom 1 | 156 | 18 | 12 |
| 8 | Hall Way | 396 | 13 | 3 | | 17 | Bedroom 3 | 396 | 53 | 13 |
| 9 | Bathroom 1 | 658 | 124 | 19 | | 17 | Landing | 604 | 53 | 9 |
| 9 | Bedroom 1 | 1782 | 133 | 7 | | 18 | Bedroom 1 | 169 | 71 | 42 |
| 9 | Office | 356 | 80 | 22 | | 18 | Bedroom 2 | 320 | 84 | 26 |
| 9 | Kitchen | 302 | 49 | 16 | | 18 | Kitchen | 187 | 49 | 26 |
| 9 | Lounge | 151 | 53 | 35 | | 18 | Lounge | 369 | 62 | 17 |
| 9 | 10 W/C | 409 | 93 | 23 | | 18 | Hall | 231 | 53 | 23 |
| 10 | Snug | 569 | 124 | 22 | | | | | | |
| 10 | Kitchen | 120 | 49 | 41 | | | | | | |
| 10 | Utility | 778 | 178 | 23 | | | | | | |

Table 5 shows the NAHA activity with and without DNJNAc added to enzyme reaction, in air samples from different rooms where some level of mould problems is seen. The relative mould index is calculated, as the NAHA activity with DNJNAc , divided by the NAHA activity without DNJNAc.

**Table 5**

| | | | NAHA activity | | Relative |
|---|---|---|---|---|---|
| | Room | | Without | With | mould |
| Building | type | | DNJNAc | DNJNAc | level % |
| 19 | Kitchen 1 | | 960 | 702 | 73 |
| 20 | Kitchen | | 2996 | 2796 | 93 |
| 20 | Bathroom | | 2147 | 1773 | 83 |
| 20 | Lounge | | 1280 | 1053 | 82 |
| 20 | Bedroom | | 1875 | 1044 | 56 |
| 20 | Hall | | 2133 | 1693 | 79 |
| 21 | Bedroom 2 | | 2760 | 1542 | 56 |
| 22 | Conservatory | | 1098 | 698 | 64 |
| 22 | Utility room | | 5560 | 5036 | 91 |
| 23 | Storage room | | 1378 | 889 | 65 |
| 23 | Bedroom | | 3140 | 2007 | 64 |
| 24 | Kitchen | | 2324 | 2071 | 89 |

Fig. 2 shows the distribution of the relative mould levels found from the data in tables 4 and 5: There is a good separation of rooms/buildings considered to be without mould problems/water damage and those to have recognized mould problems, except for one outlier (marked with an arrow in Fig. 2). The relative mould index for this particular outlier raises the suspicion that a hitherto unnoticed mould source may be present although not disclosed by the surveyor.

## Claims

1. A method for quantitative or qualitative determination of fungal cells in a sample potentially containing β-N-acetythexosaminidase3.2.1.52 (NAHA) from non-fungal sources, comprising contacting or mixing the sample with a substrate to produce a reaction mixture and subsequently assessing the numbers or concentration of the fungal cells in the sample as a function of measured conversion of the substrate in the reaction mixture after producing the reaction mixture, wherein
- the substrate can be converted by (NAHA) produced by the fungal cells and by non-fungal NAHA, and
- a preferential inhibitor of the enzymatic conversion of the substrate by non-fungal NAHA is present in the reaction mixture;
wherein the preferential inhibitor is one that upon prolonged incubation with fungal cells inhibits the NAHA activity from fungal cells in a manner similar to the inhibition of non-fungal NAHA activity,
wherein the preferential inhibitor is a metal ion selected from Ag⁺, Hg²⁺, and Pb²⁺ or is 2-Acetamido-1,2-dideoxynojirimycin.

2. A method for determination of the relative amount of fungal cells in a location compared to at least one non-fungal cell in the same location, comprising the steps of
1) contacting or mixing a sample from the location with a first substrate to produce a first reaction mixture and subsequently determining the conversion rate of the first substrate after producing the first reaction mixture,
2) contacting or mixing a sample from the location with a second substrate to produce a second reaction mixture and subsequently determining the conversion rate of the second substrate after producing the second reaction mixture, and
3) determining the relative amount of the fungal cells by calculating the ratio between the conversion rate determined in step 1 and the conversion rate determined in step 2, wherein
- the first and second substrates can be converted by both NAHA produced by the fungal cells and NAHA from non-fungal sources,
- a preferential inhibitor of the enzymatic conversion of the first substrate by NAHA from non-fungal sources is present in the first reaction mixture and a preferential inhibitor of the enzymatic conversion of the second substrate by NAHA from non-fungal sources is not present in the second reaction mixture;
wherein the preferential inhibitor is one that upon prolonged incubation with fungal cells inhibits the NAHA activity from fungal cells in a manner similar to the inhibition of non-fungal NAHA activity,
wherein the preferential inhibitor is a metal ion selected from Ag⁺, Hg²⁺, and Pb²⁺ or is 2-Acetamido-1,2-dideoxynojirimycin.

3. The method according to claim 2, wherein the first and second substrates are identical.

4. The method according to any one of the preceding claims, wherein the fungal cells are fungal particles containing NAHA, such as filamentous fungal cell(s) in the form of hyphae or spores, hyphal fragments, or hyphal microfragments having sizes less than 1 µm.

5. The method according to any one of the preceding claims, wherein the product of the conversion by NAHA is detectable and/or wherein the unconverted substrate(s) is/are detectable.

6. The method according to any one of the preceding claims, wherein conversion is measured by gauging the increase in the amount of the product of the conversion and/or the decrease in the amount of the substrate(s).

7. The method according to claim 5 or 6, wherein the detectable product(s) and/or the detectable substrate(s) is/are coloured, luminescent, fluorescent, chromogenic, enzymatically active, or a specific binding partner to a capture agent.

8. The method according to claim 7, wherein the detectable substrate(s) and/or product(s) is/are fluorescent and wherein a change in fluorescence is gauged after addition of the substrate to the sample.

9. The method according to claim 7 or 8, wherein fluorescence is gauged intermittently or continuously, preferably over a period of about 30 minutes.

10. The method according to any one of claims 7-9, wherein fluorescence is measured at one or several time points after addition of the substrate to the sample.

11. The method according to any one of the preceding claims, wherein the inhibitor preferentially inhibits conversion of the (first) substrate by non-fungal NAHA compared to the inhibition of the conversion by the fungal cells.

12. The method according to any one of the preceding claims, wherein the substrate(s) release(s) 4-methylumbelliferone or a fluorescently detectable derivative hereof when being converted by NAHA, such as a methylumbelliferyl derivative selected from the group consisting of 4-methylumbelliferyl-β-N-acetyl-D-glucosaminide, 4-methylumbelliferyl-β-D-N,N',N"-triacetylchitotrioside, 5-bromo-6-chloro-3-indolyl-2-acetamido-2-deoxy-β-D-glucopyranoside, 5-bromo-4-chloro-3-indolyl-N-acetyl-β-D-glucosaminide, indolyl-2-acetamido-2-deoxy-β-D-gluco-pyranoside, 4-nitrophenyl-N-acetyl-β-D-glucosaminide, β-trifluoromethylumbelliferyl-N-acetyl-β-D-glucosaminide, N-methylum-indolyl-N-acetyl-β-D-glucosaminide, 5-iodo-3-indolyl-N-acetyl-β-D-glucosaminide, 4-methylumbelliferyl-β-D-N,N',N"-triacetylchitotriose, 4-methylumbelliferyl-β-D-N,N'-diacetylchitobioside, 4-methylumbelliferyl-7-(6-sulfo-2-acetamido-2-deoxy)-β-D-glucosaminide, 4-methylumbelliferyl α-D-fucoside; 4-methylumbelliferyl β-D-fucoside, 4-methylumbelliferyl α-D-glucoside, 4-methylumbelliferyl-7-(6-sulfo-2-acetamido-2-deoxy-β-D-glucopyronoside), 4-methylumbelliferyl-N-acetyl-α-D-glucosaminide, 4-methylumbelliferyl β-D-lactoside, 4-methylumbelliferyl-N-acetylgalactosaminide, 4-methylumbelliferyl β-D-mannopyranoside, 4-methylumbelliferyl α-D-mannopyranoside, 4-methylumbelliferyl β-D-xyloside, resorufin-N-acetyl-β-D-glucosaminide, 4-methylumbelliferyl-N-acetyl-α-D-glucosaminide, 9H-(1,3-dichloro-9,9-dimethylacridin-2-one-7-yl)N-acetyl-β-D-glucosa minide (DDAO), and an N-actyl-β-D-glucosaminide oligomer derivative of DDAO, preferably 4-methylumbelliferyl-β-N-acetyl-D-glucosaminide and 4-methylumbelliferyl-β-D-N,N',N"-triacetylchitotrioside.

13. The method according to any one of the preceding claims, wherein the preferential inhibitor is
- mixed or contacted with the (first) substrate prior producing the (first) reaction mixture, or
- added simultaneously with the (first) substrate to the sample when producing the (first) reaction mixture, or
- added to the sample prior to producing the reaction mixture.

14. The method according to claim 13, wherein, if the inhibitor is added to the sample prior to producing the (first) reaction mixture, then the inhibitor is added to the sample at most 2 hours prior to producing the (first) reaction mixture, such as the most 115 minutes, at most 100 minutes, at most 105 minutes, at most 100 minutes, at most 95 minutes, at most 90 minutes, at most 85 minutes, at most 80 minutes, at most 75 minutes, at most 70 minutes, at most 65 minutes, at most 60 minutes, at most 55 minutes, at most 50 minutes, at most 45 minutes, at most 40 minutes, at most 35 minutes, at most 30 minutes, at most 25 minutes, at most 20 minutes, at most 15 minutes, at most 10 minutes, and at most 5 minutes prior to producing the (first) reaction mixture.

## Patentansprüche

1. Verfahren zur quantitativen oder qualitativen Bestimmung von Pilzzellen in einer Probe, die möglicherweise β-N-Acetylhexosaminidase 3.2.1.52 (NAHA) aus nicht-pilzlichen Quellen enthält, umfassend das Inkontaktbringen oder Mischen der Probe mit einem Substrat, um ein Reaktionsgemisch herzustellen und anschließendes Bestimmen der Anzahl oder Konzentration der Pilzzellen in der Probe in Abhängigkeit des gemessenen Umwandlung des Substrats in der Reaktionsmischung nach Herstellung der Reaktionsmischung, wobei
- das Substrat durch (NAHA) umgewandelt werden kann, das durch die Pilzzellen und durch nicht-pilzliches NAHA produziert wird, und
- ein bevorzugter Inhibitor der enzymatischen Umwandlung des Substrats durch nicht-pilzliches NAHA in der Reaktionsmischung vorhanden ist;
wobei der bevorzugte Inhibitor ein solcher ist, der bei längerer Inkubation mit Pilzzellen die NAHA-Aktivität von Pilzzellen in ähnlicher Weise wie die Hemmung der nicht-pilzlichen NAHA-Aktivität hemmt,
wobei der bevorzugte Inhibitor ein Metallion ist, das aus Ag⁺, Hg²⁺ und Pb²⁺ ausgewählt ist oder 2-Acetamido-1,2-dideoxynojirimycin ist.

2. Verfahren zur Bestimmung der relativen Menge an Pilzzellen an einem Ort im Vergleich zu mindestens einer nicht-pilzlichen Zelle an demselben Ort, umfassend die Schritte
1) Inkontaktbringen oder Mischen einer Probe von dem Ort mit einem ersten Substrat, um ein erstes Reaktionsgemisch herzustellen und anschließendes Bestimmen der Umwandlungsrate des ersten Substrats nach dem Herstellen des ersten Reaktionsgemischs,
2) Inkontaktbringen oder Mischen einer Probe von dem Ort mit einem zweiten Substrat, um ein zweites Reaktionsgemisch herzustellen, und anschließendes Bestimmen der Umwandlungsrate des zweiten Substrats nach dem Herstellen des zweiten Reaktionsgemischs, und
3) Bestimmen der relativen Menge der Pilzzellen durch Berechnen des Verhältnisses zwischen der in Schritt 1 bestimmten Umwandlungsrate und der in Schritt 2 bestimmten Umwandlungsrate, wobei
- das erste und zweite Substrat sowohl durch von den Pilzzellen produziertes NAHA als auch durch NAHA aus nicht-pilzlichen Quellen umgewandelt werden können,
- ein bevorzugter Inhibitor der enzymatischen Umwandlung des ersten Substrats durch NAHA aus nicht-pilzlichen Quellen in der ersten Reaktionsmischung vorhanden ist und ein bevorzugter Inhibitor der enzymatischen Umwandlung des zweiten Substrats durch NAHA aus nicht-pilzlichen Quellen in der zweiten Reaktionsmischung nicht vorhanden ist;
wobei der bevorzugte Inhibitor ein solcher ist, der bei längerer Inkubation mit Pilzzellen die NAHA-Aktivität von Pilzzellen in ähnlicher Weise wie die Hemmung der nicht-pilzlichen NAHA-Aktivität hemmt,
wobei der bevorzugte Inhibitor ein Metallion ist, das aus Ag⁺, Hg²⁺ und Pb²⁺ ausgewählt ist, oder 2-Acetamido-1,2-dideoxynojirimycin ist.

3. Verfahren nach Anspruch 2, wobei das erste und das zweite Substrat identisch sind.

4. Verfahren nach einem der vorhergehenden Ansprüche, wobei die Pilzzellen NAHA-haltige Pilzpartikel sind, wie z.B. fadenförmige Pilzzellen in Form von Hyphen oder Sporen, Hyphenfragmente oder Hyphenmikrofragmente mit einer Größe von kleiner als 1 pm.

5. Verfahren nach einem der vorhergehenden Ansprüche, wobei das Produkt der Umwandlung durch NAHA nachweisbar ist und/oder wobei das/die nicht umgewandelte(n) Substrat(e) nachweisbar ist/sind.

6. Verfahren nach einem der vorhergehenden Ansprüche, wobei die Umwandlung durch Abschätzen der Zunahme der Menge des Umwandlungsprodukts und/oder der Abnahme der Menge des/der Substrats/e gemessen wird.

7. Verfahren nach Anspruch 5 oder 6, wobei das/die nachweisbare(n) Produkt(e) und/oder das/die nachweisbare(n) Substrat(e) farbig, lumineszierend, fluoreszierend, chromogen, enzymatisch aktiv oder ein spezifischer Bindungspartner für ein Einfangmittel ist/sind.

8. Verfahren nach Anspruch 7, wobei das/die nachweisbare(n) Substrat(e) und/oder Produkt(e) fluoreszierend ist/sind und wobei eine Änderung der Fluoreszenz nach Zugabe des Substrats zu der Probe abgeschätzt wird.

9. Verfahren nach Anspruch 7 oder 8, wobei die Fluoreszenz intermittierend oder kontinuierlich, bevorzugt über einen Zeitraum von etwa 30 Minuten, abgeschätzt wird.

10. Verfahren nach einem der Ansprüche 7 bis 9, wobei die Fluoreszenz zu einem oder mehreren Zeitpunkten nach Zugabe des Substrats zu der Probe gemessen wird.

11. Verfahren nach einem der vorhergehenden Ansprüche, wobei der Inhibitor bevorzugt die Umwandlung des (ersten) Substrats durch nicht-pilzliches NAHA im Vergleich zur Hemmung der Umwandlung durch die Pilzzellen hemmt.

12. Verfahren nach einem der vorhergehenden Ansprüche, wobei das (die) Substrat(e) 4-Methylumbelliferon oder ein fluoreszierend nachweisbares Derivat davon freisetzt (freisetzen), wenn es (sie) durch NAHA umgewandelt wird (werden), wie z.B. ein Methylumbelliferylderivat, ausgewählt aus der Gruppe bestehend aus 4-Methylumbelliferyl-β-N-acetyl-D-glucosaminid, 4-Methylumbelliferyl-β-D-N,N',N"-triacetylchitotriosid, 5-Brom-6-chlor-3-indolyl-2-acetamid-2-desoxy-β-D-gluco-pyranosid, 5-Brom-4-chlor-3-indolyl-N-acetyl-β-D-glucosaminid, Indolyl-2-acetamido-2-desoxy-β-D-gluco-pyranosid, 4-Nitrophenyl-N-acetyl-β-D-glucosaminid, β-Trifluormethylumbelliferyl-N-acetyl-β-D-glucosaminid, N-Methylum-indolyl-N-acetyl-β-D-glucosaminid, 5-Iodo-3-indolyl-N-acetyl-β-D-glucosaminid, 4-Methylumbelliferyl-β-D-N,N',N"-triacetylchitotriose, 4-Methylumbelliferyl-β-D-N,N'-diacetylchitobiosid, 4-Methylumbelliferyl-7-(6-sulfo-2-acetamido-2-desoxy)-β-D-glucosaminid, 4-Methylumbelliferyl α-D-fucosid; 4-Methylumbelliferyl β-D-fucosid, 4-Methylumbelliferyl-α-D-glucosid, 4-Methylumbelliferyl-7-(6-sulfo-2-acetamido-2-desoxy-β-D-glucopyronosid), 4-Methylumbelliferyl-N-acetyl-α-D-glucosaminid, 4-Methylumbelliferyl β-D-lactosid, 4-Methylumbelliferyl-N-acetylgalactosaminid, 4-Methylumbelliferyl-D-mannopyranosid, 4-Methylumbelliferyl α-D-mannopyranosid, 4-Methylumbelliferyl β-D-xylosid, Resorufin-N-acetyl-β-D-glucosaminid, 4-Methylumbelliferyl-N-acetyl-α-D-glucosaminid, 9H-(1,3-Dichlor-9,9-dimethylacridin-2-on-7-yl)N-acetyl-β-D-glucosaminid (DDAO) und ein N-Acetyl-β-D-glucosaminid-oligomerderivat von DDAO, bevorzugt 4-Methylumbelliferyl-β-N-acetyl-D-glucosaminid und 4-Methylumbelliferyl-β-D-N,N',N"-triacetylchitotriosid.

13. Verfahren nach einem der vorhergehenden Ansprüche, wobei der bevorzugte Inhibitor
- mit dem (ersten) Substrat gemischt oder in Kontakt gebracht wird, bevor das (erste) Reaktionsgemisch hergestellt wird, oder
- beim Herstellen des (ersten) Reaktionsgemisches gleichzeitig mit dem (ersten) Substrat zu der Probe zugegeben wird, oder
- der Probe vor dem Herstellen des Reaktionsgemischs zugegeben wird.

14. Verfahren nach Anspruch 13, wobei, wenn der Inhibitor der Probe vor dem Herstellen des (ersten) Reaktionsgemisches zugegeben wird, der Inhibitor dann der Probe höchstens 2 Stunden vor der Herstellung des (ersten) Reaktionsgemisches zugegeben wird, wie z.B. höchstens 115 Minuten, höchstens 100 Minuten, höchstens 105 Minuten, höchstens 100 Minuten, höchstens 95 Minuten, höchstens 90 Minuten, höchstens 85 Minuten, höchstens 80 Minuten, höchstens 75 Minuten, höchstens 70 Minuten, höchstens 65 Minuten, höchstens 60 Minuten, höchstens 55 Minuten, höchstens 50 Minuten, höchstens 45 Minuten, höchstens 40 Minuten, höchstens 35 Minuten, höchstens 30 Minuten, höchstens 25 Minuten, höchstens 20 Minuten, höchstens 15 Minuten, höchstens 10 Minuten und höchstens 5 Minuten vor dem Herstellen der (ersten) Reaktionsmischung.

## Revendications

1. Procédé de détermination quantitative ou qualitative de cellules fongiques dans un échantillon contenant potentiellement de la β-N-acétylhexosaminidase 3.2.1.52 (NAHA) provenant de sources non fongiques, comprenant la mise en contact ou le mélange de l'échantillon avec un substrat pour produire un mélange réactionnel et ensuite l'évaluation du nombre ou de la concentration des cellules fongiques dans l'échantillon en fonction d'une conversion mesurée du substrat dans le mélange réactionnel après la production du mélange réactionnel, dans lequel
- le substrat peut être transformé par la (NAHA) produite par les cellules fongiques et par la NAHA non fongique, et
- un inhibiteur préférentiel de la conversion enzymatique du substrat par la NAHA non fongique est présent dans le mélange réactionnel ;
dans lequel l'inhibiteur préférentiel est un inhibiteur qui, lors d'une incubation prolongée avec des cellules fongiques, inhibe l'activité de la NAHA des cellules fongiques d'une manière similaire à l'inhibition de l'activité de la NAHA non fongique,
dans lequel l'inhibiteur préférentiel est un ion métallique choisi parmi Ag⁺, Hg²⁺ et Pb²⁺ ou est la 2-acétamido-1,2-didésoxynojirimycine.

2. Procédé de détermination de la quantité relative de cellules fongiques dans un emplacement par rapport à au moins une cellule non fongique dans le même emplacement, comprenant les étapes
1) de mise en contact ou de mélange d'un échantillon provenant de l'emplacement avec un premier substrat pour produire un premier mélange réactionnel et ensuite de détermination du taux de conversion du premier substrat après la production du premier mélange réactionnel,
2) de mise en contact ou de mélange d'un échantillon provenant de l'emplacement avec un second substrat pour produire un second mélange réactionnel et ensuite de détermination du taux de conversion du second substrat après la production du second mélange réactionnel, et
3) de détermination de la quantité relative des cellules fongiques en calculant le rapport entre le taux de conversion déterminé à l'étape 1 et le taux de conversion déterminé à l'étape 2, dans lequel
- les premier et second substrats peuvent être convertis à la fois par la NAHA produite par les cellules fongiques et par la NAHA provenant de sources non fongiques,
- un inhibiteur préférentiel de la conversion enzymatique du premier substrat par la NAHA provenant de sources non fongiques est présent dans le premier mélange réactionnel et un inhibiteur préférentiel de la conversion enzymatique du second substrat par la NAHA provenant de sources non fongiques n'est pas présent dans le second mélange réactionnel ;
dans lequel l'inhibiteur préférentiel est un inhibiteur qui, lors d'une incubation prolongée avec des cellules fongiques, inhibe l'activité de la NAHA des cellules fongiques d'une manière similaire à l'inhibition de l'activité de la NAHA non fongique,
dans lequel l'inhibiteur préférentiel est un ion métallique choisi parmi Ag⁺, Hg²⁺ et Pb²⁺ ou est la 2-acétamido-1,2-didésoxynojirimycine.

3. Procédé selon la revendication 2, dans lequel les premier et second substrats sont identiques.

4. Procédé selon l'une quelconque des revendications précédentes, dans lequel les cellules fongiques sont des particules fongiques contenant de la NAHA, telles qu'une ou des cellules fongiques filamenteuses sous forme d'hyphes ou de spores, de fragments d'hyphes ou de microfragments d'hyphes ayant des tailles inférieures à 1 pm.

5. Procédé selon l'une quelconque des revendications précédentes, dans lequel le produit de la conversion par la NAHA est détectable et/ou dans lequel le ou les substrats non convertis sont détectables.

6. Procédé selon l'une quelconque des revendications précédentes, dans lequel la conversion est mesurée en mesurant l'augmentation de la quantité du produit de la conversion et/ou la diminution de la quantité du ou des substrat(s).

7. Procédé selon la revendication 5 ou 6, dans lequel le ou les produits détectables et/ou le ou les substrats détectables sont colorés, luminescents, fluorescents, chromogènes, actifs sur le plan enzymatique, ou un partenaire de liaison spécifique à un agent de capture.

8. Procédé selon la revendication 7, dans lequel le ou les substrats et/ou le ou les produits détectables sont fluorescents et dans lequel un changement de fluorescence est mesuré après addition du substrat à l'échantillon.

9. Procédé selon la revendication 7 ou 8, dans lequel la fluorescence est mesurée de manière intermittente ou continue, de préférence sur une durée d'environ 30 minutes.

10. Procédé selon l'une quelconque des revendications 7 à 9, dans lequel la fluorescence est mesurée à un ou plusieurs instants après addition du substrat à l'échantillon.

11. Procédé selon l'une quelconque des revendications précédentes, dans lequel l'inhibiteur inhibe préférentiellement la conversion du (premier) substrat par la NAHA non fongique par rapport à l'inhibition de la conversion par les cellules fongiques.

12. Procédé selon l'une quelconque des revendications précédentes, dans lequel le ou les substrats libèrent de la 4-méthylumbelliférone ou un dérivé détectable par fluorescence de celle-ci lorsqu'ils sont convertis par la NAHA, tel qu'un dérivé de méthylumbelliféryle choisi dans le groupe constitué de 4-méthylumbelliféryl-β-N-acétyl-D-glucosaminide, 4-méthylumbelliféryl-β-D-N,N',N"-triacétylchitotrioside, 5-bromo-6-chloro-3-indolyl-2-acétamido-2-désoxy-β-D-glucopyranoside, 5-bromo-4-chloro-3-indolyl-N-acétyl-β-D-glucosaminide, indolyl-2-acétamido-2-désoxy-β-D-glucopyranoside, 4-nitrophényl-N-acétyl-β-D-glucosaminide, β-trifluorométhylumbelliféryl-N-acétyl-β-D-glucosaminide, N-méthylum-indolyl-N-acétyl-β-D-glucosaminide, 5-iodo-3-indolyl-N-acétyl-β-D-glucosaminide, 4-méthylumbelliféryl-β-D-N,N',N"-triacétylchitotriose, 4-méthylumbelliféryl-β-D-N,N'-diacétylchitobioside, 4-méthylumbelliféryl-7-(6-sulfo-2-acétamido-2-désoxy)-β-D-glucosaminide, 4-méthylumbelliféryl α-D-fucoside ; 4-méthylumbelliféryl-β-D-fucoside, 4-méthylumbelliféryl-α-D-glucoside, 4-méthylumbelliféryl-7-(6-sulfo-2-acétamido-2-désoxy-β-D-glucopyronoside), 4-méthylumbelliféryl-N-acétyl-α-D-glucosaminide, 4-méthylumbelliféryl-β-D-lactoside, 4-méthylumbelliféryl-N-acétylgalactosaminide, 4-méthylumbelliféryl-β-D-mannopyranoside, 4-méthylumbelliféryl-α-D-mannopyranoside, 4-méthylumbelliféryl-β-D-xyloside, résorufine-N-acétyl-β-D-glucosaminide, 4-méthylumbelliféryl-N-acétyl-α-D-glucosaminide, 9H-(1,3-dichloro-9,9-diméthylacridin-2-one-7-yl)N-acétyl-β-D-glucosaminide (DDAO), et un dérivé oligomère de N-actyl-β-D-glucosaminide de DDAO, de préférence le 4-méthylumbelliferyl-β-N-acétyl-D-glucosaminide et le 4-méthylumbelliferyl-β-D-N,N' ,N"-triacétylchitotrioside.

13. Procédé selon l'une quelconque des revendications précédentes, dans lequel l'inhibiteur préférentiel est
- mélangé ou mis en contact avec le (premier) substrat avant la production du (premier) mélange réactionnel, ou
- ajouté simultanément avec le (premier) substrat à l'échantillon lors de la production du (premier) mélange réactionnel, ou
- ajouté à l'échantillon avant la production du mélange réactionnel.

14. Procédé selon la revendication 13, dans lequel, si l'inhibiteur est ajouté à l'échantillon avant la production du (premier) mélange réactionnel, alors l'inhibiteur est ajouté à l'échantillon au plus 2 heures avant la production du (premier) mélange réactionnel, comme au plus 115 minutes, au plus 100 minutes, au plus 105 minutes, au plus 100 minutes, au plus 95 minutes, au plus 90 minutes, au plus 85 minutes, au plus 80 minutes, au plus 75 minutes, au plus 70 minutes, au plus 65 minutes, au plus 60 minutes, au plus 55 minutes, au plus 50 minutes, au plus 45 minutes, au plus 40 minutes, au plus 35 minutes, au plus 30 minutes, au plus 25 minutes, au plus 20 minutes, au plus 15 minutes, au plus 10 minutes et au plus 5 minutes avant la production du (premier) mélange réactionnel.
